# EUROPEAN PATENT APPLICATION

(11) **EP 4 450 122 A1**
(43) Date of publication of application: **23.10.2024**
(21) Application number: 23168993.6
(22) Date of filing: 20.04.2023
(51) Int. Cl.: A61N 1/36, A61N 1/04

(54) **SYSTEM FOR ELECTRICALLY STIMULATING A REGION LOCATED IN A BRAIN**

(71) Applicant: Université d'Aix Marseille, 13007 Marseille (FR); Institut National de la Santé et de la Recherche Médicale, 75013 Paris (FR)
(72) Inventor: MISSEY, Florian, 13004 MARSEILLE (FR); BOTZANOWSKI, Boris, 13010 MARSEILLE (FR); WILLIAMSON, Adam, 13010 MARSEILLE (FR); ACERBO, Emma, 13010 MARSEILLE (FR)
(74) Representative: Macquet, Christophe

(57) **Abstract**

The invention relates to a deep brain stimulation system and method for electrically stimulating a region located in a brain of a mammal, comprising a first and a second pair of stimulation electrodes capable of delivering a stimulating electric alternating current at a second frequency of at least 800 Hz and at a second phase; a difference between the first carrier frequency and the second carrier frequency being of at least 0.5 Hz; the first phase being approximately equal to the second phase; at least a first pair of anti-stimulation electrodes capable of delivering an electric alternating current at a carrier frequency substantially equal to the first carrier frequency or the second carrier frequency or substantially equal to a multiple of these first or second carrier frequencies, but having a phase shift of approximately 180° as compared to the first and/or second phase.

## Description

### FIELD OF THE INVENTION

The invention relates to a deep brain stimulation system for electrically stimulating a region located in a brain of a mammal. It also relates to a method for performing a deep brain stimulation of a region located in a brain of a mammal.

### BACKGROUND OF THE INVENTION

Brain stimulation methods use electromagnetic fields to interfere with the brain activity. These methods may be invasives or non-invasives. The degree of invasiveness and the focality of the delivered stimulations are two key distinctions between these methods.

Non-invasive brain stimulation, such as Transcranial Magnetic Stimulation (TMS) and transcranial Direct or Alternating Current Stimulations (tDCS and tACS), gained increasing popularity in recent years due to their great potential in treating various psychiatric alterations such as: disorders of depression spectrum and Obsessive-compulsive Disorders (OCDs). However, transcranial techniques are generally employed to target cortical structures because they have low focality for deep brain regions such as the striatum and subthalamic nuclei.

Invasive brain stimulation techniques, such as deep brain stimulation (DBS), involve the placement of electrodes into the brain. Kindled by the therapeutic usage of DBS in Parkinson patients at the second half of 20th century, DBS is currently expanded to the treatment of various psychiatric disorders such as OCDs, depression, addiction and Tourette. However, compared to non-invasive brain stimulation, DBS requires a surgical intervention that is linked with a higher risk of complications, such as infection, bleeding, and damage to surrounding brain tissue. At the same time, invasive techniques are generally more precise and effective in targeting specific brain regions and circuits.

The choice between invasive and non-invasive techniques depends on the specific needs of the patient and the condition being treated. In some cases, non-invasive techniques may be sufficient to achieve the desired therapeutic effect, while, in other cases, invasive techniques may be necessary to achieve the required precision and effectiveness.

In recent years, it was developed a non-invasive brain stimulation (NIBS) protocol called Temporal Interference stimulation (TI), that allows the stimulation of a subcortical area, the hippocampus. TI relies on the use of topical electrodes placed directly on the animal/patient scalp. In the case of a dipolar TI, two electric stimuli are delivered through these electrodes. For instance, the waveform of the electric stimuli and the amplitude can be selected depending on-demand as long as the frequency of these carriers' field is higher than 1 kHz. As the waves are travelling in the brain, they overlap and interfere in a desired region in which the destructive and additive interference of the stimuli creates a third frequency in the Hz range. Finally, only the difference in frequency field is causing neurons to depolarize at depth with a limited cortical activation.

TI stimulation was originally designed having for two current sources with source electrodes placed on the skull and return on the body. This design was later modified to put all electrodes from two current sources on the skull, i.e. a dipole configuration. Over the last years, TI was used in animal and human-based studies, demonstrating the stimulation of cortical and subcortical brain areas. Numerous studies have demonstrated the usage of TI with two interfering field for a stimulation of deep brain areas: hippocampus and superior colliculus in mice but also mesial-temporal lobe and striatum in humans, as well as non-brain structures such as sciatic nerve and bladder.

However, a recent computational modelling study conducted an in-depth analysis of the TI biophysics and exposed the presence of previously unreported off-target TI stimulations. In addition, this in-depth analysis explained why the focality assessments made in previous TI studies did not detect the model-predicted non-Region-of-Interest (non-RoI) stimulations. In other studies, off-target stimulations were not explicitly measured. However, it was shown, using computational modelling, that, upon the increase of TI stimulation amplitude, the TI envelope extends and may activate off-target structures with a lower activation threshold. Similar off-target stimulation issues are known to exist with other electrical stimulation paradigms, such as TMS, transcranial Electrical Stimulation (tES), or Vagus Nerve Stimulation (VNS).

In order to improve the focality of the stimulation, and avoid stimulation of unwanted zones, it has been proposed to increase the number of stimulators. This technique relies on the addition of several envelopes having the maximum amplitude of envelope at the same spot. Such invention is not published on the filing on the filing date of this patent application.

### SUMMARY OF THE INVENTION

Accordingly, a need exists for improving the focality of the stimulation, and for avoiding stimulation of unwanted zones in a brain of a mammal, when implementing deep brain stimulation.

In accordance with a first aspect, the invention concerns a deep brain stimulation system for electrically stimulating a region located in a brain of a mammal, comprising
a first pair of stimulation electrodes capable of delivering a stimulating electric alternating current at a first carrier frequency of at least 800 Hz and at a first phase;
a second pair of stimulation electrodes capable of delivering a stimulating electric alternating current at a second carrier frequency of at least 800 Hz and at a second phase;
a difference between the first carrier frequency and the second carrier frequency being of at least 0.5 Hz;
the first phase being approximately equal to the second phase; and
at least a first pair of anti-stimulation electrodes capable of delivering an electric alternating current at a carrier frequency substantially equal to the first carrier frequency or the second carrier frequency or substantially equal to a multiple of these first or second carrier frequencies, but having a phase shift of approximately 180° as compared to the first and second phase.

Preferentially, - the system further comprises a third pair of stimulation electrodes capable of delivering a stimulating electric alternating current at a third carrier frequency of at least 800 Hz and at a third phase; a fourth pair of stimulation electrodes capable of delivering a stimulating electric alternating current at a fourth carrier frequency of at least 800 Hz and at a fourth phase; a difference between the third carrier frequency and the fourth carrier frequency being of at least 0.5 Hz; a difference between the mean frequencies of the first and second pairs of electrodes and the mean frequencies of the third and fourth pairs of electrodes being of at least 200 Hz; - the system further comprises at least a second pair of anti-stimulation electrodes capable of delivering an electric alternating current at a carrier frequency substantially equal to the third carrier frequency or the fourth carrier frequency or substantially equal to a multiple of these third or fourth carrier frequencies, but having a phase shift of approximately 180° as compared to the third and/or fourth phase; - the difference between the first carrier frequency and the second carrier frequency or between the third carrier frequency and the third carrier frequency is comprised between 10 Hz and 150 Hz, preferably between 25 and 75 Hz; - the difference of the mean frequencies of, on one hand, a first couple of stimulation pairs of electrodes and, on the other hand, a second couple of stimulation pairs of electrodes, is of at least 500 Hz and even, preferably of at least 800 Hz, for example 900 Hz; - the voltage of the alternating current delivered by the pair of anti-stimulation electrodes is less than the voltage of the alternating current delivered by the pair of stimulation electrodes; and - the electrodes are capable of being positioned on a surface of a scalp of a mammal for a non-invasive deep brain stimulation of the brain of the mammal.

In accordance with a second aspect, the invention concerns a method for performing a deep brain stimulation of a region located in a brain of a mammal, comprising
providing a first pair of stimulation electrodes delivering a stimulating electric alternating current at a first carrier frequency of at least 800 Hz and at a first phase;
providing a second pair of stimulation electrodes delivering a stimulating electric alternating current at a second carrier frequency of at least 800 Hz and at a second phase;
a difference between the first carrier frequency and the second carrier frequency being of at least 0.5 Hz;
the first phase being approximately equal to the second phase; and
providing at least a first pair of anti-stimulation electrodes capable of delivering an electric alternating current at a carrier frequency substantially equal to the first carrier frequency or the second carrier frequency or substantially equal to a multiple of these first or second carrier frequencies, but having a phase shift of approximately 180° as compared to the first and/or second phase.

Preferentially, - the method further comprises providing a third pair of stimulation electrodes capable of delivering a stimulating electric alternating current at a third carrier frequency of at least 800 Hz and at a third phase; providing a fourth pair of stimulation electrodes capable of delivering a stimulating electric alternating current at a fourth carrier frequency of at least 800 Hz and at a fourth phase; a difference between the third carrier frequency and the fourth carrier frequency being of at least 0.5 Hz; a difference between the mean frequencies of the first and second pairs of electrodes and the mean frequencies of the third and fourth pairs of electrodes being of at least 200 Hz; the method further comprises providing at least a second pair of anti-stimulation electrodes capable of delivering an electric alternating current at a carrier frequency substantially equal to the third carrier frequency or the fourth carrier frequency or substantially equal to a multiple of these third or fourth carrier frequencies, but having a phase shift of approximately 180° as compared to the third and/or fourth phase; and - the stimulation is non-invasive.

### BRIEF DESCRIPTION OF THE DRAWINGS

Other features and aspects of the present invention will be apparent from the following description and the accompanying drawings, in which:
Fig. 1 illustrates the principle of phase cancelation implemented in the invention;
Fig. 2 illustrates the focality gain which derives from the phase cancelling Temporal Interference stimulation according to the invention;
Fig. 3 illustrates a system according to the invention comprising four pairs of stimulation electrodes and two pairs anti-electrodes;
Fig. 4A illustrates the gain in focality that may be obtained by implementing the method according to the invention, in the case where the TI stimulation is achieved using two pairs of stimulation electrodes;
Fig. 4B illustrates the gain in focality that may be obtained by implementing the method according to the invention, in the case where the TI stimulation is achieved using four pairs of stimulation electrodes;
Figs. 5A, 5B and 5C illustrates the computational modelling that has been implemented for evaluating the advantages of the invention over the state of the art. In particular Fig. 5A illustrates a highly segmented mouse model with stimulation electrodes (N=38) positioned in accordance with high-density EEG system. Fig. 5B show the ten electrode configurations with the best focality values for stimulation of IL area. In Fig. 5C, it is shown the TI field generated with AF3, AF7 and AF4, AF8 electrode configuration that have the highest focality;
Figs. 6A and 6B compares a temporal interference in Infralimbic Regions (IL) and Motor Cortex (MC) using a dipole configuration (Fig. 6A) or using a tripole configuration according to the invention; and
Fig. 7 illustrates that phase cancelling according to the method of the invention improves the stimulation focality.

### DETAILLED DESCRIPTION OF THE INVENTION

Temporal Interference (TI) allows deep brain stimulation that gather the level of the deepness of regular deep brain stimulation protocols and the non-invasiveness of transcranial magnetic electric stimulation. TI stimulation allows researchers or clinicians to stimulate deep brain structure without damaging the brain. However, as TI stimulation focuses on one deep target in the brain, the whole brain receives TI fields and other areas could possibly be stimulated with off-target stimulation.

The invention proposes an alternative to TI protocols, that reduces off-target stimulation without reducing the TI field in the desired focus.

It relates to a system and a method for performing a deep brain stimulation of a region located in a brain of a mammal, in particular a human brain, that implements a phase cancelation.

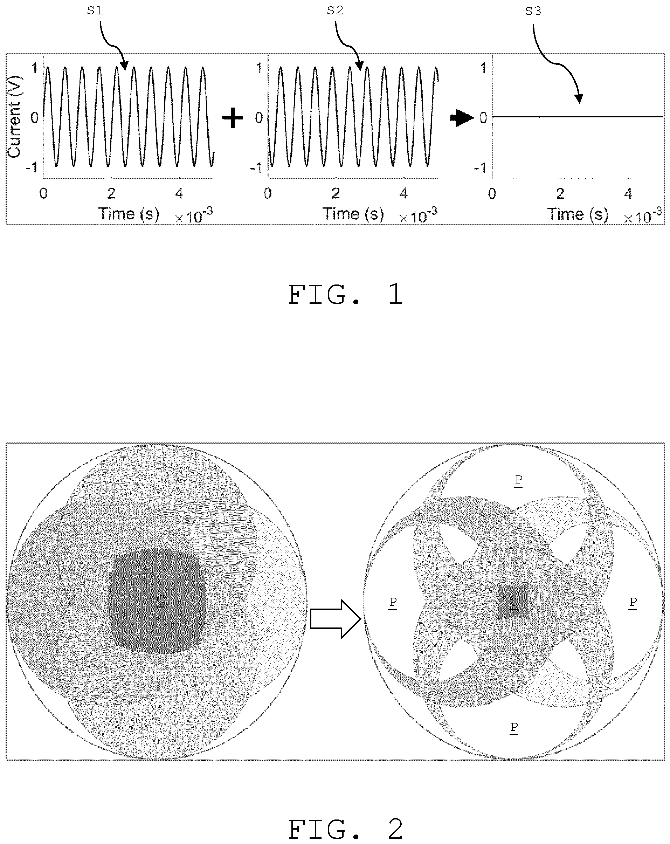

The principle of phase cancelation is illustrated in Fig. 1. As appearing in Fig. 1, phase cancelation occurs when two electric signals S1, S2 of the same frequency but of opposite phase interact. Such interaction leads to a reduced or a cancelled signal S3.

As illustrated in Fig. 2, the invention exploits phase cancelation to reduce the stimulation effectiveness in shallower portions P of brain tissues while retaining the desired efficiency at depth, at a targeted zone C, and also improving focality of stimulation in this zone C. In order to implement the invention, an anti-stimulator is positioned, for example, next to each stimulator or next to one stimulator. An ideal position would be, for the stimulator and its anti-stimulator, to coexist in the same location, but as current sources need to be isolated, it is not practically possible. The anti-stimulator delivers the same or substantially the same frequency as its stimulator but at opposite charge and, advantageously, at a lower voltage. As the current delivered by the anti-stimulator is lower, it will travel less deep than the one from the stimulator into the brain, and will then dampen the signal from the main stimulator in the shallower portion. On the left side of Fig. 2, a TI quadrupole, i.e. comprising four pairs of electrodes, is illustrated and, on the right side, a same TI quadrupole is illustrated with anti-electrodes according to the invention. While the amplitude of all electrodes is the same in both cases, the area C, where the stimulation is relevant, is significantly reduced when phase cancelling is applied. The focality of stimulation in the zone C is improved.

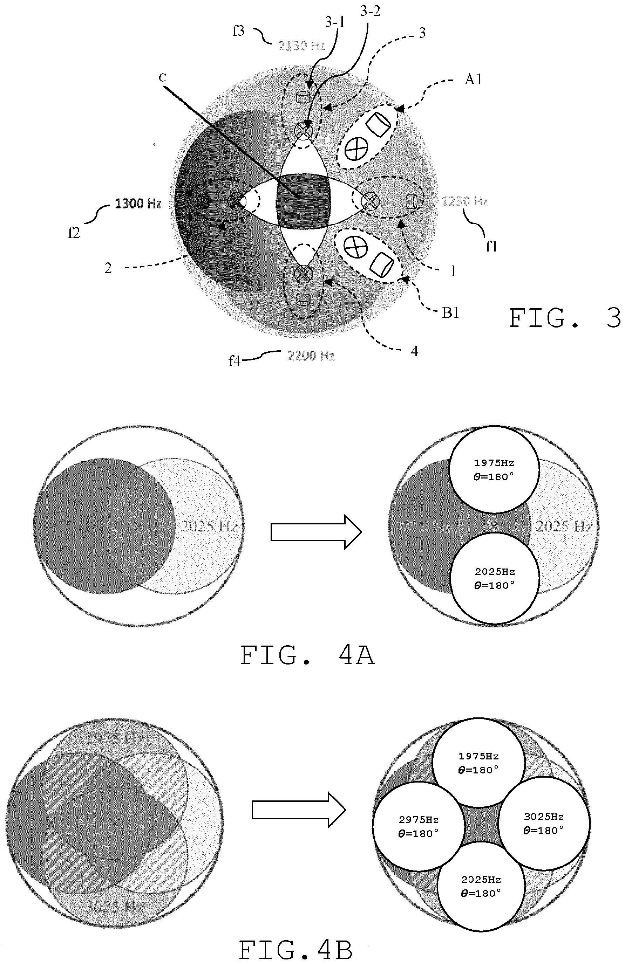

An example of a deep brain stimulation system for electrically stimulating a region located in a brain of a mammal is illustrated in Fig. 3. This system comprises a first pair 1 of stimulation electrodes capable of delivering a stimulating electric alternating current at a first carrier frequency f1 of at least 800 Hz, for example 1250 Hz, and according to a first phase. The pair of electrodes is a couple of electrodes that are illustrated by a cylinder and a circle comprising a cross in Fig. 3. These electrodes are referenced for example 3-1 and 3-2 in such figure. The system also comprises a second pair 2 of stimulation electrodes capable of delivering a stimulating electric alternating current at a second frequency f2 of at least 800 Hz, for example 1300 Hz, and according to a second phase. The difference between the first carrier frequency f1 and the second carrier frequency f2 is of at least 10 Hz. In the example of Fig. 3, it is of 50 Hz. Advantageously, the system according to the invention comprises a third pair 3 of stimulation electrodes capable of delivering a stimulating electric alternating current at a third carrier frequency f3 of at least 800 Hz, for example 2150 Hz, and at a third phase. It also comprises a fourth pair 4 of stimulation electrodes capable of delivering a stimulating electric alternating current at a fourth carrier frequency f4 of at least 800 Hz, for example 2200 Hz, and at a fourth phase. The difference between the third carrier frequency f3 and the fourth carrier frequency f4 is of at least 10 Hz. In the example of Fig. 3, it is of 50 Hz. The first phase is equal or approximately equal to the second phase. In other words, there is no shift between the phases of the first and second electrode currents. It is the same for the third and the fourth electrode currents. The difference between the mean frequencies of the first 1 and second 2 pairs of electrodes and the mean frequencies of the third 3 and fourth 4 pairs of electrodes is of at least 200 Hz. It is for example of 900 Hz.

The system according to the invention also comprises at least a first pair A1 of anti-stimulation electrodes capable of delivering an electric alternating current at a carrier frequency substantially equal to the first carrier frequency or the second carrier frequency or substantially equal to a multiple of these first or second carrier frequencies, but having a phase shift of approximately 180° as compared to the first and second phase. It may comprise more than one first pair A1 of anti-stimulation electrodes. The first pair of anti-stimulation electrodes are positioned, for example, at the vicinity of the first pair of stimulation electrodes.

The system comprises at least a second pair B1 of anti-stimulation electrodes capable of delivering an electric alternating current at a carrier frequency substantially equal to the third carrier frequency or the fourth carrier frequency or substantially equal to a multiple of these third or fourth carrier frequencies, but having a phase shift of approximately 180° as compared to the third and fourth phase. The number of anti-stimulation electrodes may be decoupled from the number of stimulation electrodes. For instance, for a system comprising 2 pairs of stimulation electrodes, there may be 1 or 2 pairs of anti-stimulating electrodes. For a system comprising 4 pairs of stimulation electrodes, there may be 1, 2, 3 or 4 pairs of anti-stimulation electrodes.

Thus, the system implements stimulation electrodes in pairs, and at least one anti-stimulating electrodes pair. As shown in Fig. 4A, there are 2 stimulation electrode pairs defining a dipole. However, there may be advantageously 4 stimulation electrode pairs that define a quadrupole, as shown in Fig. 4B. In other modes for carrying out the invention, the number of pairs will be more than 4, for example equal to 8, which will then form an octupole.

The stimulation electrode pairs are positioned on the surface of the scalp, around the head of a mammal, for instance a human individual. The position of the electrodes of a pair of electrodes may be close one to another, even at an immediate vicinity, or at various positions, for instance, at opposite positions around the patient's head. However, the positioning of the electrodes should advantageously be calculated beforehand in such a way as to have a maximum modulation at the point of interest within the brain, while minimizing the electric current delivered by each electrode. If the situation allows it, positioning electrodes on various axes, such as the axes anterior-posterior, medio-lateral or dorsa-ventral, should result in a greater focal gain.

It is also to be noted that the electrodes should not act as capacitors during stimulation in order to avoid non-faradaic processes. Typical Electrocardiogram (ECG) electrodes, which are composed of Ag/AgCl or electrodes coated with Poly(3,4-ethylenedioxythiophene) (PEDOT)-Poly(styrene sulfonate) (PSS) may be used according to the invention.

Any signal generator may be implemented in the system according to the invention, as long as it can generate high frequencies as disclosed herein, and it is possible to modify the phase of the signal. Generally, the generator is connected to one or more electric current sources. The current sources themselves electrically connected to the electrodes. Each pair of electrodes is connected to an individual signal source and the highest stimulation intensity is then concentrated between the electrodes of each such pair. The current sources are independent.

On a general point of view, it will be noted that:
advantageously, the carrier frequency of a stimulation pair of electrodes is of at least 800 Hz, preferentially of at least 900 Hz, and more preferentially of at least 1000 Hz;
advantageously, the difference between the carrier frequencies of two stimulating pairs of electrodes that are associated is of at least 0.5 Hz, preferably between 10 Hz and 150 Hz, and more preferably between 25 and 75 Hz, for example equal or approximately equal to 50 Hz.
advantageously, in order to avoid unwanted stimulation areas, the difference of the mean frequencies of, on one hand, a first couple of stimulation pairs of electrodes and, on the other hand, a second couple of stimulation pairs of electrodes, is of at least 200 Hz, preferably of at least 500 Hz and even more preferably of at least 800 Hz, for example 900 Hz.

In a mode for carrying out the invention, a tripole configuration is proposed for TI in which one of the three pairs will stimulate with an active phase cancellation, with a 180° phase shift, to target Infralimbic Regions (IL) in mice. It is shown a significant decrease in rogue TI fields in IL-neighboring areas. The tripole configuration addresses one of the disadvantages of TI by efficiently reducing off-target stimulation and thus avoiding non-wanted stimulation of brain structures that might trigger epileptic seizures of cognitive disorders.

For the experimental study, the IL of an anesthetized mice was stimulated using a dipole TI stimulation. Off-target stimulations were directly evaluated by measuring Local Field Potentials (LFPs), which subsequently suggested that the inclusion of an additional stimulation pair with an active phase shift of 180 degrees could eliminate them, hence increasing the focality.

The TI stimulation was used with a dipole configuration to stimulate a deep brain structure, namely the IL, which is highly implicated in cognitive processing and psychiatric diseases. To highlight the residual TI stimulation in off-target areas, LFPs were directly measured in IL neighboring regions (bilateral recordings of hippocampi and motor cortex). As expected, rogue stimulation outside of the targeted area was found and using the newly proposed tripole TI configuration could efficiently reduce this adverse effect.

To determine the optimal electrode configuration for targeting the IL using TI stimulation, a modeling pipeline was devised to assess the efficacy of TI under various experimental setups. The pipeline consisted of three stages, namely (i) the construction of a head model with electrodes, (ii) the simulation of the electric fields, (iii) and comparison of the resulting TI fields. The pipeline was implemented using Sim4Life^{™}, a simulation platform developed by Zurich Med Tech AG, Switzerland. This software was previously used in initial studies that provided evidence for the effectiveness of TI in a petri dish/phantom and a mouse model.

First, it was aimed to determine the best configuration of the electrode location to target the IL with TI stimulation. The coordinates of the target ROI (IL) were based on Allen Brain Common Coordinate Framework. Using the Sim4Life^{™} software, it was simulated TI fields within the mouse brain under different placements for the two electrode pairs (e.g. dipole) on the scalp. Overall, 38 electrodes were placed on the brain to be tested as potential stimulation electrode or ground. To assess the efficacy of these TI fields for targeting the IL, two parameters were computed for each stimulation threshold and focality. The threshold corresponded to the median TI field calculated within the IL. The focality corresponded to the ratio between the volume activated higher than the threshold in the IL and the whole brain. Finally, the database of TI fields was created with the corresponding threshold and focality values. This database was sorted for focality and the TI field with the highest threshold was chosen as an optimal one.

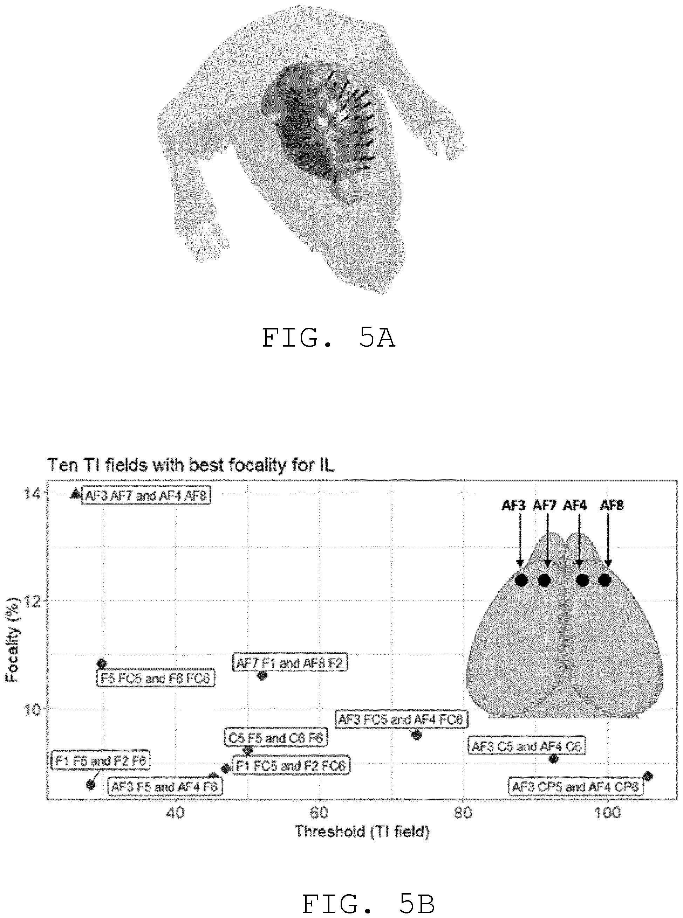

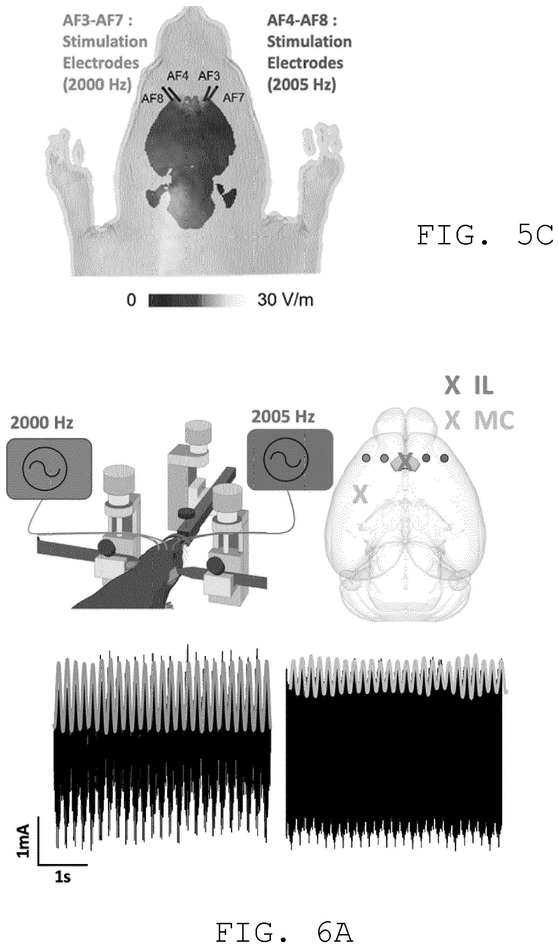

Fig. 5A illustrates a highly segmented mouse model with stimulation electrodes (N=38) positioned in accordance with high-density EEG system. In Fig. 5B, 10 electrode configurations are shown with the best focality values for stimulation of IL area. In Fig. 5C, the TI field generated with AF3, AF7 and AF4, AF8 electrode configuration had the highest focality. The electrodes are shown in dark gray, while the IL target is highlighted in light gray on the heatmap.

Two approaches can be used to steer the TI field to the region of interest: (i) changing the weights of the electric fields and (ii) changing the locations of electrodes. While the first approach may be advantageous for lateral brain targets, it is not practical when the region of interest is located in the middle of the brain, as in this investigation. This is because the electric fields generated with equal weights have an intercept with the TI amplitude modulation that peaks at central areas of the brain, and the TI field amplitude is expected to be highest under the condition of the equal weight. Therefore, the second approach was employed to search for an efficient TI field, and the stimulation was compared under different electrode configurations.

For this approach, however, the number of simulations to be computed resulted in 221 445 of possible combinations with 38 electrodes. Since the objective of the pipeline was to determine a suitable electrode configuration for a specific target in the brain, rather than exhaustively exploring the entire sample space, an additional limitation was imposed to reduce the number of combinations to be calculated. This limitation involved focusing on combinations that exclusively consisted of symmetrical electrode pairs relative to the sagittal slice (such as AF3 and AF4), as it was observed that these configurations had the highest TI field peak in the center of the brain where the IL is located. By selecting only 18 symmetrical pairs, the number of combinations decreased to 153 TI fields, which were then computed and compared in terms of their ability to target the IL region.

To assess the efficacy of the TI fields in targeting the IL, two parameters were computed: threshold and focality. The threshold parameter was determined as the median TI field value calculated within the IL, while the focality parameter was estimated as the ratio between the number of voxels activated higher than the threshold in the target and the other parts of the brain. These parameters can be interpreted using the following rationale: higher threshold values correspond to stronger expected TI field strengths within the target, while greater focality values indicate a more concentrated field within the region of interest in comparison to the entire brain.

The aforementioned parameters were estimated for each TI field in the collected database, and then sorted for focality (Fig 5B). As the results of these steps, it was determined an optimal TI field to stimulate the IL area, which was generated with 2 pairs of electrodes, AF3 to AF7 and AF4 to AF8 (Fig 5C), and according to the simulation results will have a threshold value in IL of 26.15 V/m under 1 mA peak-to-baseline stimulation. The predicted focality of this configuration, which was the highest among the collected fields, equals to 13.95%.

Importantly, the model predicted the induction of TI field in the areas close and distant from IL: motor cortex (6.5 V/m) (Fig 5B) which is, relative to TI electric field in IL, way less but could still elicit some subthreshold activity both in the motor cortex.

The used computational model assumes the location of the electrodes only on the scalp, without considering electrodes that might touch the cortex. To ensure that TI could be provided both with electrodes on top of the cortex and on top of the scalp, these two approaches are tested in a rodent model. Therefore, at the next step it was aimed to check the model predictions by in-vivo experiments.

To estimate the induction of TI stimulation outside of IL region, according to the Allen Atlas, for the above-described dipole configuration LFP recordings were performed in anesthetized mice (Fig. 6A). The TI induction was measured in a region distal to the IL by recording LFP from the IL and left primary motor cortex. For TI stimulation, a 5µA alternating current (AC) was applied to each of two independent current sources producing sine waves at either 2000 or 2005 Hz (Fig. 6A), thereby inducing an amplitude modulation at the frequency of 5Hz.

By recording stimulation artifacts directly in the IF region, it could be highlighted that the low-frequency amplitude modulation (5 Hz) indeed results from TI stimulation (Fig. 6A). TI amplitude in IL and MOp were also compered to estimate the induction of the field outside of RoI (Fig. 6A). As expected by the model predictions (Fig. 5B) the TI amplitude in IL was the highest compared to MOp (Fig. 6A) and decreased along the AP axis.

In conclusion, it is demonstrated that using a dipole arrangement can induced TI stimulation in the IL region. TI was, however, passively induced in distant and off-target brain areas. Even with optimal electrode placements and current ratios, it was showed that passive TI stimulation cannot be abolished by reducing the amplitudes of TI-inducing fields. The combined results of dipole TI stimulation of IL show the need for further improvement in focality.

As TI amplitude is dependent on the vector sum of the applied fields, one of the key ideas underlying the focality enhancement was that the interference spot produced by many fields will have stronger amplitude modulation (e.g. the TI amplitude) than the interference produced by two fields. Additionally, the produced fields will have a shift in their phases when they arrive at the interference spot due to the different spatial locations of the stimulation electrodes on the skull. As a result, the cancellation occurred at the areas with a phase shift between the fields of the same frequency. This phase shift is comprised between 170 and 190°. It is preferentially of 180°. To increase the focality of our approach, it was decided to introduce an additional pair of electrodes that would produce a field 180 degrees shifted (i.e. an antiphase field) with respect to the stimulation fields of our original dipole configuration. Employing an active phase shift - a phase shift produced by the function generator - and an anatomy driven position of the cancellation pair, one may increase the focality of TI without requiring symmetrical electrode placement, and potentially minimizing the number of electrodes.

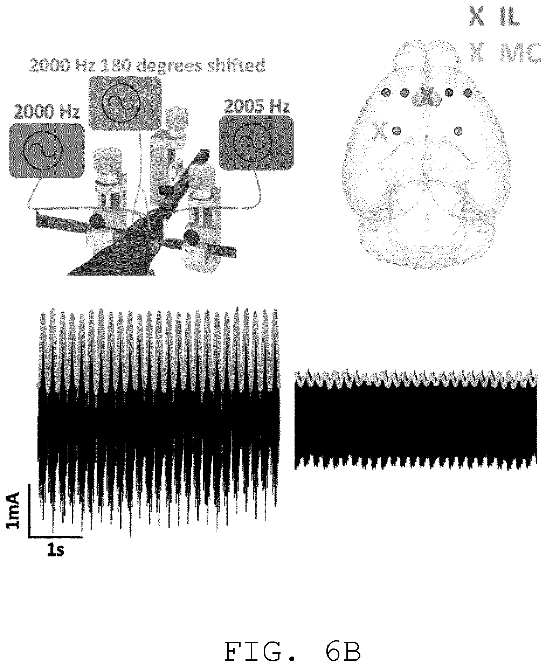

To validate the cancellation according to the invention, the first objective was to cancel TI stimulation in a single brain area. Motor cortex (Mop) was chosen as a target brain region and introduced cancelling field as shown in Fig. 6B. The cancelling field was generated by introducing an additional independent current source with the same frequency as one of the stimulation fields (i.e., 2000Hz), but phase-shifted of 180 degrees compared to both stimulation fields (i.e. 2000 and 2005Hz). Using COMSOL^{™} software the TI field was computer in the MOp area with different amplitudes of the cancelling field (cancelling weights). The modeling results were checked by measuring MOp LFPs in anaesthetized mice (e.g. in-vivo) upon the application of various cancelling weights.

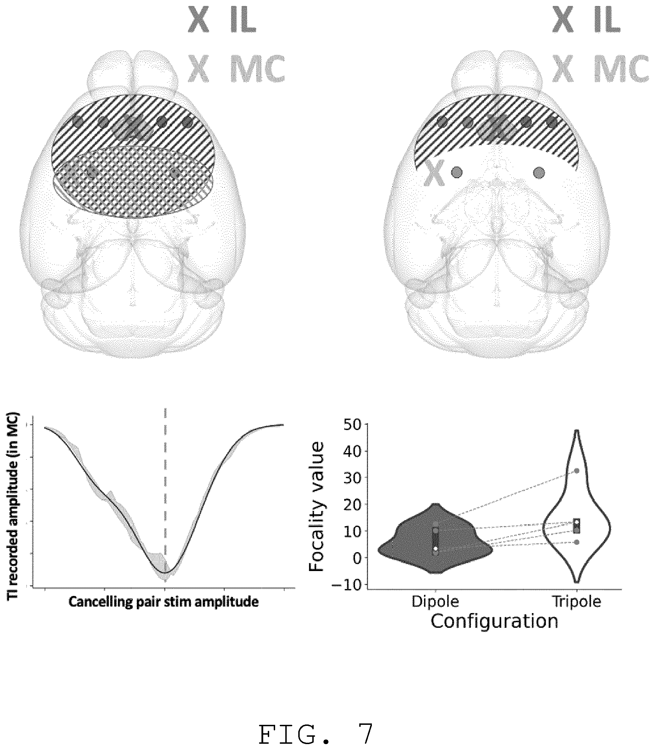

A stepwise increase of the canceling weight led to a cancellation curve in MOp in both in-silico and in-vivo setups (Fig. 7). The obtained cancellation curve shown in Fig. 7 is the result of two co-occurring processes: "cancellation" (Left side of the curve) and "side induction" (Right side of the curve). Cancellation refers to the destruction of the temporal interference by a canceling field, whereas side induction attributes to temporal interference occurring between the cancellation field and one of the stimulation fields. Specifically, side cancellation occurs between a cancellation field and a stimulation field with which the cancellation field has a shift in frequency. Upon an initial increase of the canceling weight, TI amplitude in MOp decreased due to cancellation (Fig. 6B). However, with a further increase of the canceling weight, a larger portion of the cancellation field interfered with one of the stimulation fields, therefore, boosting side induction. The cancellation curve was symmetrical with respect to the minimum TI amplitude and was well-fitted with a double Gaussian curve at the group level (Fig. 7) and in individual animals. The symmetry of the cancellation curve suggests that the processes driving the results on either side of the curve are similar, further validating the mechanism of cancellation we proposed above.

Finally, TI stimulation can lead to off-target stimulation that can be harmful depending on the location and intensity of this unwanted stimulation. TI stimulation was described in mice brain through TI artifact recording and its propagation rather than actual neuronal activity (neuronal spiking) to emphasize how the focality of TI stimulation can be improved. Furthermore, it was aimed to focus on the infralimbic region in mice which is analogous to the ventromedial prefrontal cortex (vmPFC) in humans and is highly implicated in both cognitive processes and psychiatric disorders making him a target of choice for the implementation of TI in further human studies. The in-vivo LFP recordings for the in-silico predicted optimal dipole TI stimulation in mouse infralimbic (IL) area exposed that non-target brain region (MC - Motor Cortex), also, showed statistically significant activation with amplitudes comparable to IL.

A tripole configuration with an additional stimulation pair that has an active (e.g., source-driven) 180-degree phase shift was used in the provided stimulation. With this tripole configuration, the activation can be selectively decreased in the non-target brain area (Motor Cortex), not significantly affecting RoI (infralimbic). The proposed mechanistic explanation of the cancellation effect suggests that by considering (i) the geometric location and (ii) brain anisotropy, one can implement the active phase canceling in multipair designs. For instance, one can shape the resulting interference spot and prioritize the absence of the off-target stimulations in a specific brain area, without physically changing electrode locations. Alternatively, if a brain area where the stimulation should be absent is fixed, a single canceling pair can be located close to this area, not requiring symmetrical organization of the multipair. Moreover, a tripole design with the addition of a cancelling electrode pair will actively cancel unwanted stimulation along an axis (Fig. 7) but an alternative using a single electrode would also be efficient is the cancellation is not needed along a specific target. In that case, the cancellation will occur as circular equipotential lines around the cancelling electrode.

Tripole configuration for TI off-target stimulation opens new perspectives for ultra-focused TI in both animal and human studies. However, the added cancellation pair should be tuned in such a way that (i) the orientation of the cancellation electric field does not affect the target TI stimulation but also (ii) the amplitude needs to be set so that no new rogue TI fields are induced in between the cancellation pair and one of the two dipole pairs. This limitation of cancellation TI again showcases the advantages of an in silico preparation that should encounter, in advance, for every parameter of the TI (dipole or tripole) protocols such as the position of each electrode pair, the amplitude of each applied electric current and the phase tuning if necessary.

Off-target stimulation being one of the most harmful disadvantages of electric stimulation in the nervous system, suppressing or at least reducing it creates great benefits for later implementation of TI protocols in clinic, for example, for investigating brain networks. It is believed that the proposed active phase cancellation concept and its mechanistic explanation provide promising insights for improving the focality of TI stimulation and can be integrated into the existing TI but also tACS protocols.

The invention thus proposes a practical approach for selectively cancelling TI stimulation in a particular area of the brain and outlined the potential mechanism for this cancellation process.

## Claims

1. A deep brain stimulation system for electrically stimulating a region located in a brain of a mammal, comprising
a first pair (1) of stimulation electrodes capable of delivering a stimulating electric alternating current at a first carrier frequency (f1) of at least 800 Hz and at a first phase;
a second pair (2) of stimulation electrodes capable of delivering a stimulating electric alternating current at a second carrier frequency (f2) of at least 800 Hz and at a second phase;
a difference between the first carrier frequency (f1) and the second carrier frequency (f2) being of at least 0.5 Hz;
the first phase being approximately equal to the second phase;
at least a first pair (A1) of anti-stimulation electrodes capable of delivering an electric alternating current at a carrier frequency substantially equal to the first carrier frequency (f1) or the second carrier frequency (f2) or substantially equal to a multiple of these first or second carrier frequencies, but having a phase shift of approximately 180° as compared to the first and/or second phase.

2. The system of claim 1, further comprising
a third pair (3) of stimulation electrodes capable of delivering a stimulating electric alternating current at a third carrier frequency (f3) of at least 800 Hz and at a third phase;
a fourth pair (4) of stimulation electrodes capable of delivering a stimulating electric alternating current at a fourth carrier frequency (f4) of at least 800 Hz and at a fourth phase;
a difference between the third carrier frequency (f3) and the fourth carrier frequency (f4) being of at least 0.5 Hz;
a difference between the mean frequencies (f1, f2) of the first and second pairs of electrodes and the mean frequencies (f3, f4) of the third and fourth pairs of electrodes being of at least 200 Hz.

3. The system of one of the claims 1 or 2, further comprising at least a second pair (A2) of anti-stimulation electrodes capable of delivering an electric alternating current at a carrier frequency substantially equal to the third carrier frequency (f3) or the fourth carrier frequency (f4) or substantially equal to a multiple of these third or fourth carrier frequencies, but having a phase shift of approximately 180° as compared to the third and/or fourth phase.

4. The system of one of the claims 1, 2 or 3, wherein the difference between the first carrier frequency (f1) and the second carrier frequency (f2) or between the third carrier frequency (f3) and the third carrier frequency (f4) is comprised between 10 Hz and 150 Hz, preferably between 25 and 75 Hz.

5. The system of one of the claims 2, 3 or 4, wherein the difference of the mean frequencies of, on one hand, a first couple of stimulation pairs of electrodes (1, 2) and, on the other hand, a second couple of stimulation pairs of electrodes (3, 4), is of at least 500 Hz and even, preferably of at least 800 Hz, for example 900 Hz.

6. The system of one of the claims 1 to 6, wherein, the voltage of the alternating current delivered by the pair (A1, B1) of anti-stimulation electrodes is less than the voltage of the alternating current delivered by the pair (1, 2, 3, 4) of stimulation electrodes.

7. The system of one of the claims 1 to 6, wherein the electrodes are capable of being positioned on a surface of a scalp of a mammal for a non-invasive deep brain stimulation of the brain of the mammal.

8. A method for performing a deep brain stimulation of a region located in a brain of a mammal, comprising
providing a first pair (1) of stimulation electrodes delivering a stimulating electric alternating current at a first carrier frequency (f1) of at least 800 Hz and at a first phase;
providing a second pair (2) of stimulation electrodes delivering a stimulating electric alternating current at a second carrier frequency (f2) of at least 800 Hz and at a second phase;
a difference between the first carrier frequency (f1) and the second carrier frequency (f2) being of at least 0.5 Hz;
the first phase being approximately equal to the second phase;
providing at least a first pair (A1) of anti-stimulation electrodes capable of delivering an electric alternating current at a carrier frequency substantially equal to the first carrier frequency or the second carrier frequency or substantially equal to a multiple of these first or second carrier frequencies, but having a phase shift of approximately 180° as compared to the first and/or second phase.

9. The method of claim 8, further comprising providing a third pair (3) of stimulation electrodes capable of delivering a stimulating electric alternating current at a third carrier frequency (f3) of at least 800 Hz and at a third phase;
providing a fourth pair (4) of stimulation electrodes capable of delivering a stimulating electric alternating current at a fourth carrier frequency (f4) of at least 800 Hz and at a fourth phase;
a difference between the third carrier frequency (f3) and the fourth carrier frequency (f4) being of at least 0.5 Hz;
a difference between the mean frequencies (f1, f2) of the first and second pairs of electrodes and the mean frequencies (f3, f4) of the third and fourth pairs of electrodes being of at least 200 Hz.

10. The method of one of the claims 8 or 9, further comprising providing at least a second pair (A2) of anti-stimulation electrodes capable of delivering an electric alternating current at a carrier frequency substantially equal to the third carrier frequency (f3) or the fourth carrier frequency (f4) or substantially equal to a multiple of these third or fourth carrier frequencies, but having a phase shift of approximately 180° as compared to the third and/or fourth phase.

11. The method of one of the claims 8 to 10, wherein the stimulation is non-invasive.
